# EUROPÄISCHE PATENTSCHRIFT

(11) **EP 1 695 953 B1**
(45) Veröffentlichungstag und Bekanntmachung des Hinweises auf die Patenterteilung: **27.05.2015**
(21) Anmeldenummer: 06100080.8
(22) Anmeldetag: 04.01.2006
(51) Int. Cl.: C07C 17/16, C07C 19/01, B01J 31/18

(54) **Verfahren zur Herstellung von Alkylchloriden**
Process for the preparation of alkyl chlorides
Procédé de préparation de chlorures d'alkyle

(30) Priorität: 23.02.2005 DE 102005008547
(43) Veröffentlichungstag der Anmeldung: 30.08.2006
(73) Patentinhaber: Evonik Degussa GmbH, 45128 Essen (DE)
(72) Erfinder: Osterholt, Clemens, 46286, Dorsten (DE); Neumann, Manfred, 45770, Marl (DE); Kübelbäck, Thomas, 48249, Dülmen (DE); Bodmann, Kerstin, 45772, Marl (DE)

(56) Entgegenhaltungen:
- WO-A-2005/026089
- DE-A1- 10 158 376
- DATABASE CA [Online] CHEMICAL ABSTRACTS SERVICE, COLUMBUS, OHIO, US; NAKAO, MASAAKI ET AL: "Alkyl halides" XP002383692 gefunden im STN Database accession no. 1978:405900 -& JP 53 015303 A (IPPOSHA OIL INDUSTRIES CO., LTD., JAPAN) 13. Februar 1978 (1978-02-13)
- DATABASE CASREACT [Online] CHEMICAL ABSTRACTS SERVICE, COLUMBUS, OHIO, US; TANG, JIE ET AL.: "Method for converting primary alcohol or cyclohexanol into alkyl halide in acidic ion liquid [Hmin]+X- (X = Cl, Br, I)" XP002383693 gefunden im STN Database accession no. 143:229439 & CN 1 440 958 A (CENTRAL-CHINA NORMAL UNIV; EAST CHINA NORMAL UNIVERSITY) 10. September 2003 (2003-09-10)
- WU, HAI-HONG; SUN, JING; YANG, FAN; TANG, JIE; HE, MING-YUAN: "Immobilization of HX: [Hmim]X as halogenating agent, recyclable catalyst, and medium for conversion of alcohols to alkyl halides" CHINESE JOURNAL OF CHEMISTRY., Bd. 22, 2004, Seiten 619-621, XP008064950 XXXX
- JURSIC B: "ORGANIC SYNTHESIS IN MICELLAR MEDIA. OXIDATION OF ALCOHOLS AND THEIR CONVERSION INTO ALKYL CHLORIDES" SYNTHESIS, GEORG THIEME VERLAG, STUTTGART, DE, Nr. 11, 1. November 1988 (1988-11-01), Seiten 868-871, XP000025117 ISSN: 0039-7881

## Beschreibung

Die Erfmdung betrifft ein Verfahren zur Herstellung von Alkylchloriden durch Umsetzung der entsprechenden Alkohole mit gasförmigem Chlorwasserstoff in Gegenwart eines Katalysators.

Die Herstellung von acyclischen Chlorkohlenwasserstoffen aus den entsprechenden Alkoholen durch Umsetzung mit Chlorwasserstoff gehört zu den allgemein gängigen Methoden der organischen Chemie und wurde bereits im "Traité de Chimie organique" von Grignard und Baud (Paris 1935, Band III, Seite 249) beschrieben.

Gemäß dem Stand der Technik wird diese Reaktion unter Verwendung eines Katalysators durchgeführt. Neben Lewis-Säuren, insbesondere in Form des Chlorids des Eisens, Aluminiums, Arsens, Antimons, Zinns oder Zinks, werden auch Aminhydrohalogenide, vor allem Aminhydrobromide und -chloride, eingesetzt. Bei diesen Aminen handelt es sich beispielsweise um primäre, sekundäre oder tertiäre Amine mit unverzweigten oder verzweigten acyclischen oder cyclischen Kohlenstoffketten oder um Amine mit aromatischen Gruppen. Insbesondere kann es sich auch um heteroaromatische Amine, wie beispielsweise Alkylpyridine, handeln.

EP 0 789 013, DE 101 58 376 und DE 102 47 497 beschreiben ein kontinuierliches Verfahren zur Herstellung von Alkylchloriden durch Umsetzung von Alkoholen mit Chlorwasserstoff in Gegenwart eines Katalysators, wie beispielsweise einer wässrigen Alkylpyridinhydrochlorid-Lösung.

Das Derwent-Abstract zu JP 53-015303 beschreibt die Herstellung von Alkylhalogeniden durch Umsetzung von Alkoholen, die mindestens 4 Kohlenstoffatome aufweisen, mit einer wässrigen Lösung von Halogenwasserstoff in Gegenwart von quaternären Ammoniumverbindungen als Katalysator. Chemical Abstracts zur JP 53-015303 beschreibt beispielhaft die Herstellung des Katalysators Stearylpyridiniumbromid, indem eine Mischung von 1 mol Stearylbromid und 1 mol Pyridin bei einer Temperatur von 80 bis 100°C für 5 Stunden gerührt wird. Zur Herstellung von Laurylchlorid wird eine Mischung aus Laurylalkohol und konzentrierter Salzsäure für 24 Stunden bei erhöhter Temperatur erhitzt. Jursic beschreibt in Synthesis 11 (1988), 868-871 die Umsetzung von primären Alkoholen mit wässriger Salzsäure in Anwesenheit von Micellen (als Phasenvermittler), wie beispielsweise Hexadecyltrimethylammoniumbromid und Hexadecylpyridiniumbromid (Cetylpyridiniumbromid). Die Isolierung der gebildeten Alkylchloride erfolgt durch aufwändige Extraktion mit Petrolether, Filtration über Kieselgel und anschließender Destillation im Vakuum. Das als Beispiel angegebene Octylchlorid wird dabei lediglich mit einer Reinheit von 96% erhalten. WO 2005/026089 A2 beschreibt die Herstellung von Halogenalkanen aus Alkoholen und Halogenwasserstoff unter Verwendung ionischer Flüssigkeiten als Katalysatoren, zum Beispiel N-Alkylpyridiumsalzen oder N-Alkylimidazoliumsalzen.

Aufgabe der vorliegenden Erfindung war es, ein optimiertes technisches Verfahren zur Herstellung von Alkylchloriden bereitzustellen, das sich durch kurze Reaktionszeiten, höhere Raum-Zeit-Ausbeuten, sowie hohe Umsätze bezogen auf den eingesetzten Alkohol auszeichnet. Insbesondere war es die Aufgabe der vorliegenden Erfindung die Aufarbeitung und Reinigung sowie die Lagerung des eingesetzten Katalysators gegenüber den Verfahren gemäß dem Stand der Technik zu vereinfachen.

Überaschenderweise wurde gefunden, dass bei Einsatz von Katalysatoren, die Verbindungen gemäß der Struktur **I** aufweisen, die Umsetzung von Alkoholen und Diolen mit gasförmigem Chlorwasserstoff gegenüber Verfahren gemäß dem Stand der Technik, die Pyridiniumhydrochlorid als Katalysator verwenden, mit deutlich kürzeren Reaktionszeiten bei einem hohen Umsatz bezogen auf den eingesetzten Alkohol erfolgt. Auf diese Weise wird ein kontinuierliches Verfahren zur Herstellung von Alkylchloriden mit hohen Raum-Zeit-Ausbeuten zugänglich. Das erfindungsgemäße Verfahren hat den Vorteil, dass diese Herstellung der Alkylchloride in gängigen technischen Anlagen umgesetzt werden kann. Gegenüber den Verfahren gemäß dem Stand der Technik, die Alkylpyridinhydrochlorid als Katalysator einsetzen, hat das erfindungsgemäße Verfahren den Vorteil, dass der Schmelzpunkt des eingesetzten Katalysators deutlich niedriger ist, so dass auf den Einsatz einer wässrigen Lösung des Katalysators und dem Beheizen von Anlagenbauteilen mit Ausnahme des Reaktors- wie es gemäß dem Stand der Technik notwendig ist - verzichtet werden kann. Besonderer Vorteil des erfindungsgemäßen Verfahrens ist, dass der Katalysator nahezu unabhängig von dem eingesetzten Alkohol eingesetzt werden kann. Dies bedeutet, dass die Alkylgruppe am Stickstoffatom des Katalysators in dem erfindungsgemäßen Verfahren ausreichend stabil ist, so dass die Alkylgruppe R₁ am Stickstoffatom in Struktur **I** nicht identisch mit der Alkylgruppe des Alkohols bzw. des herzustellenden Alkylchlorids sein muss. Dadurch kann ein und derselbe Katalysatortyp in dem erfindungsgemäßen Verfahren für die

Herstellung von verschiedenen Alkylchloriden eingesetzt werden. Durch den Einsatz von gasförmigem Chlorwasserstoff kann die anfallende Abwassermenge bei dem erfindungsgemäßen Verfahren deutlich gegenüber den Verfahren gemäß dem Stand der Technik, die mit wässriger Salzsäure arbeiten, verringert werden. Das erfindungsgemäße Verfahren hat ferner den Vorteil, dass durch den Verzicht auf Katalysatoren gemäß der Struktur **I** mit Bromid- statt Chlorid-Anionen keine Gefahr besteht, dass sich neben den erwünschten Alkylchloriden auch Alkylbromide bilden.

Gegenstand der Erfindung ist ein Verfahren zur Herstellung von Alkylchloriden durch Umsetzung der entsprechenden Alkohole mit gasförmigem Chlorwasserstoff in Gegenwart eines Katalysators, welches sich dadurch auszeichnet, dass ein Katalysator, der zumindest eine Verbindung gemäß der Struktur I 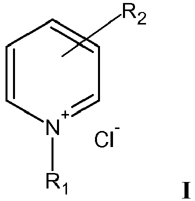 mit
- R₁:: lineare Alkylgruppe mit einer Anzahl an Kohlenstoffatomen von 1 bis 20,
- R₂:: Alkyl-Gruppe mit einer Anzahl an Kohlenstoffatomen von 1 bis 20,
aufweist, in Form einer Mischung eingesetzt wird, die verschiedene Isomere an mono-alkylierten und/oder mehrfach alkylierten N-Alkylpyridiniumchloriden aufweist, wobei sich die mehrfach alkylierte N-Alkylpyridiniumchloride durch mehrere Substituenten vom Typ R₂ am Stickstoffheterocyclus auszeichnen.

Insbesondere eignet sich das erfindungsgemäße Verfahren zur Herstellung von Alkylchloriden, die von 3 bis 20, bevorzugt von 4 bis 14 und besonders bevorzugt von 4 bis 8 Kohlenstoffatome aufweisen. Unter Alkylchloriden werden im Sinne dieser Erfindung sowohl Monochloralkane als auch Dichloralkane, insbesondere α,ω-Dichloralkane, verstanden. In einer besonderen Ausführungsform des erfindungsgemäßen Verfahrens werden Dichloralkane, die bevorzugt von 3 bis 20, besonders bevorzugt von 4 bis 12 und ganz besonders bevorzugt von 4 bis 8 Kohlenstoffatome aufweisen, hergestellt.

Die in dem erfindungsgemäßen Verfahren eingesetzten Katalysatoren weisen zumindest eine Verbindung gemäß der Struktur **I** auf, wobei diese Verbindung bevorzugt einen Schmelzpunkt von maximal 100°C aufweist. Salze der Struktur **I,** die einen Schmelzpunkt von maximal 100°C aufweisen, werden in der Regel als ionische Flüssigkeiten bezeichnet.

In dem erfindungsgemäßen Verfahren wird ein Katalysator eingesetzt, der zumindest eine Verbindung gemäß der Struktur **II** aufweist: 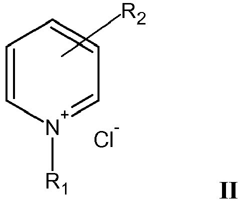 mit
- R₁:: lineare Alkylgruppe mit einer Anzahl an Kohlenstoffatomen von 1 bis 20,
- R₂:: Alkyl-Gruppe mit einer Anzahl an Kohlenstoffatomen von 1 bis 20.

Unter einer Alkylaryl-Gruppe wird im Sinne dieser Erfindung eine durch Alkylgruppen substituierte Aryl-Gruppe, wie beispielsweise H₃C-CH₂-C₆H₄-, und unter einer Aralkyl-Gruppe eine durch Aryl-Gruppen substituierte Alkylgruppe, wie beispielsweise H₅C₆-CH₂-CH₂-, verstanden.

Besonders bevorzugt wird in dem erfindungsgemäßen Verfahren ein Katalysator eingesetzt, der zumindest eine Verbindung gemäß der Struktur **II** aufweist, wobei der Substituent vom Typ R₁ vorzugsweise von 2 bis 13 Kohlenstoffatome, bevorzugt von 3 bis 8 Kohlenstoffatome aufweist. Besonders bevorzugt wird in dem erfindungsgemäßen Verfahren ein Katalysator eingesetzt, der zumindest eine Verbindung gemäß der Struktur **II** aufweist, die als Substituenten vom Typ R₁ eine n-Butyl- oder eine n-Octyl-Gruppe, insbesondere eine n-ButylGruppe aufweist. Die Katalysatoren, die eine Verbindung gemäß der Struktur **II** aufweisen, weisen als Substituenten vom Typ R₂ eine Alkylgruppe, bevorzugt mit einer

Anzahl an Kohlenstoffatomen von 1 bis 8, bevorzugt von 2 bis 4, auf.

Insbesondere wird in dem erfindungsgemäßen Verfahren ein Katalysator eingesetzt, der N-n-Butyl-alkylpyridiniumchlorid gemäß der Struktur **III** mit einem Substituenten vom Typ R₂, ausgewählt aus Methyl- oder Ethyl-Gruppe, aufweist:

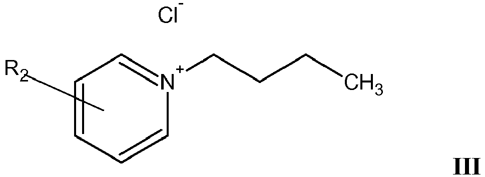

In dem erfindungsgemäßen Verfahren werden vorzugsweise Katalysatoren eingesetzt, die N-n-Octyl-alkylpyridiniumchlorid gemäß der Struktur **IV** mit einem Substituenten R₂, ausgewählt aus Methyl- oder Ethyl-Gruppe, aufweisen:

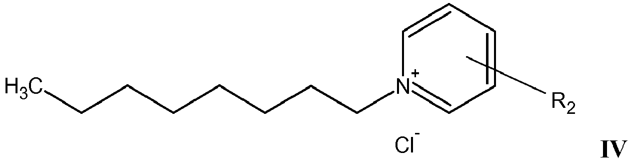

Im erfindungsgemäßen Verfahren wird als Katalysator eine Mischung eingesetzt, die verschiedene Isomere an alkylierten N-Alkylpyridiniumchloriden aufweist. Besonders bevorzugt weist die als Katalysator eingesetzte Mischung verschiedene Isomere an N-Alkylmethylpyridiumchloriden und/oder N-Alkylethylpyridiniumchloriden auf. Insbesondere weist der eingesetzte Katalysator eine Mischung bestehend aus N-Alkyl-2-methylpyridiumchlorid, N-Alkyl-3-methylpyridiniumchlorid, N-Alkyl-4-methylpyridiniumchlorid und/oder N-Alkyl-2-Ethylpyridiniumchlorid auf. Neben den mono-alkylierten N-Alkylpyridiniumchloriden kann die als Katalysator eingesetzte Mischung auch mehrfach alkylierte N-Alkylpyridiniumchloride aufweisen, die sich durch mehrere Substituenten vom Typ R₂ am Stickstoffheterocyclus auszeichnen. Als Katalysator wird eine Mischung eingesetzt, die verschiedene Isomere an mono-alkylierten und/oder mehrfach alkylierten N-Alkylpyridiniumchloriden aufweist, wobei sich die mehrfach alkylierte N-Alkylpyridiniumchloride durch mehrere Substituenten vom Typ R₂ am Stickstoffheterocyclus auszeichnen. Die in dem erfindungsgemäßen Verfahren als Katalysator eingesetzten Mischungen können neben den alkylierten N-Alkylpyridiniumchloriden auch Alkyl- und/oder Dialkylpyridine, insbesondere 2-Methylpyridin, 3-Methylpyridin, 4-Methylpyridin, 2-Ethylpyridin, 2,3-Dimethylpyridin, 2,4-Dimethylpyridin, 2,5-Dimethylpyridin, 2,6-Dimethylpyridin, aufweisen. Insbesondere wird hierbei der eingesetzte Katalysator in einem vorgeschalteten Verfahrensschritt hergestellt.

Insbesondere wird in dem erfindungsgemäßen Verfahren ein Katalysator eingesetzt, der N-n-Butyl-alkylpyridiniumchlorid gemäß der Struktur **III** mit einem Substituenten vom Typ R₂, ausgewählt aus Methyl- oder Ethyl-Gruppe, aufweist. Besonders bevorzugt weist der Katalysator eine Mischung aus verschiedenen Isomeren des N-n-Butyl-alkylpyridiniumchlorids auf, wobei diese Mischung auch mehrfach alkylierte N-n-Butylpyridiniumchloride enthalten kann. Diese mehrfach alkylierten N-n-Butylalkylpyridiniumchloride zeichnen sich durch mehrere Substituenten vom Typ R₂ am Stickstoffheterocyclus aus.

Als Edukte können in dem erfindungsgemäßen Verfahren Alkohole mit einer oder zwei Hydroxy-Gruppen eingesetzt werden. Bevorzugt wird ein Alkohol eingesetzt, der von 3 bis 20, besonders bevorzugt von 4 bis 14 und ganz besonders bevorzugt von 4 bis 8 Kohlenstoffatome aufweist. Der eingesetzte Alkohol oder das eingesetzte Diol kann linear oder verzweigt sein. Vorzugsweise wird in dem erfindungsgemäßen Verfahren ein linearer Alkohol mit einer Hydroxy-Gruppe eingesetzt, bevorzugt ausgewählt aus n-Propanol, n-Butanol, n-Hexanol, n-Octanol, n-Tetradecanol, n-Octadecanol. In einer weiteren Ausführungsform des erfindungsgemäßen Verfahrens wird ein verzweigter Alkohol, insbesondere mit einer Hydroxygruppe, bevorzugt ausgewählt aus iso-Propanol, iso-Butanol, sec-Butanol, 2-Ethylhexanol, eingesetzt. In einer weiteren Ausführungsform werden in dem erfindungsgemäßen Verfahren Diole - Alkohole mit zwei Hydroxy-Gruppen - eingesetzt, bevorzugt werden α,ω-Dihydroxyalkane und besonders bevorzugt werden Diole, ausgewählt aus 1,6-Hexandiol, 1,8-Octandiol und 1,10-Decandiol, eingesetzt.

Das erfindungsgemäße Verfahren kann als batch-, als semikontinuierliches oder als ein kontinuierliches Verfahren durchgeführt werden, insbesondere wird es als semikontinuierliches oder kontinuierliches Verfahren durchgeführt. Bevorzugt wird das erfindungsgemäße Verfahren kontinuierlich, insbesondere in einem Reaktor und besonders bevorzugt in mehreren Reaktoren, die in Form einer Kaskade mit einander verbunden sind, durchgeführt. Besonders bevorzugt wird das erfindungsgemäße Verfahren in einer Kaskade mit mindesten zwei Reaktoren durchgeführt. Die Herstellung von Alkylchloriden mit einer Anzahl von Kohlenstoffatomen von 11 bis 20 wird vorzugsweise als semikontinuierliches Verfahren durchgeführt.

Die bei dem erfmdungsgemäßen Verfahren entstehenden Reaktionsprodukte werden vorzugsweise dampfförmig ohne oder über eine aufgesetzte Kolonne abgezogen und kondensiert. Hierbei wird vorzugsweise das bei der Reaktion sich gebildete Wasser entweder azeotrop mit dem gebildeten Alkylchlorid oder direkt destillativ abgetrennt. Die sich bildenden Phasen werden getrennt und einer separaten Aufarbeitung zugeführt. Die untere wässrige Phase kann über eine Stripperkolonne von den organischen Bestandteilen befreit werden, diese können anschließend der Reaktion erneut zugeführt werden. Die obere organische Phase wird vorzugsweise teilweise als Kolonnenrückfluss eingesetzt, die übrige Menge kann zur Entfernung von restlichem Alkohol alkalisch extrahiert und über Ätznatron getrocknet werden. Wird hierbei bereits eine Reinheit des Alkylchlorids von über 99% erreicht, so kann eine Destillation entfallen.

Das Reaktionsprodukt kann in dem erfindungsgemäßen Verfahren über einen seitlich angebrachten Überlauf abgetrennt werden. Vorzugsweise wird zur Abtrennung des Reaktionsproduktes eine Teilmenge der Reaktionsmischung, beispielsweise über einen seitlichen Überlauf, entnommen und zur Phasentrennung in einen Ruhebehälter überführt werden. Die Katalysatorphase - die untere Phase - kann abgetrennt und in die Reaktion zurückgeführt werden. Die obere Produktphase kann je nach Art des Alkylchlorids der weiteren Aufarbeitung, z.B. einer Wäsche und/oder Destillation, zugeführt werden. Diese Verfahrensvariante eignet sich bevorzugt bei der Herstellung von höher- bzw. hochsiedenden Alkylchloriden, die von 7 bis 20 Kohlenstoffatome aufweisen, insbesondere von Alkylchloriden, die von 7 bis 12 Kohlenstoffatome aufweisen.

In einer weiteren Ausführungsform des erfindungsgemäßen Verfahren wird das Reaktionsprodukt dampfförmig aus dem Reaktor ausgetragen, insbesondere erfolgt dies mittels Azeotropbildung mit Salzsäure. Anschließend wird das Produkt kondensiert und mittels Phasentrennung von dem Reaktionswasser getrennt. Die erhaltene organische Phase kann anschließend mittels thermischen Trennverfahren, insbesondere Destillation, aufgearbeitet werden. Diese Verfahrensvariante eignet sich bevorzugt für niedrigsiedende Alkylchloride, die von 3 bis 10 Kohlenstoffatome aufweisen, insbesondere für Alkylchloride, die von 4 bis 8 Kohlenstoffatome aufweisen.

In einer weiteren Ausführungsform des erfindungsgemäßen Verfahren wird das bei der azeotropen Destillation entfernte Zielprodukt - Alkylchlorid - nach der Phasentrennung in den Reaktor zurückgeführt, vorteilhafterweise erfolgt dies an einem Wasserauskreiser.

Das erfindungsgemäße Verfahren wird insbesondere bei einer Temperatur von 60 bis 160°C durchgeführt. Für die Herstellung von Alkylchloriden mit einer Anzahl von Kohlenstoffatomen von 3 bis 6 wird das erfindungsgemäße Verfahren bevorzugt bei einer Temperatur von 60 bis 160°C, besonders bevorzugt von 80 bis 150°C durchgeführt, während die Herstellung von Alkylchloriden mit einer Anzahl von Kohlenstoffatomen von 7 bis 10 bevorzugt bei einer Temperatur von 110 bis 160°C, besonders bevorzugt von 130 bis 150°C durchgeführt wird. Bei einer Temperatur von 100 bis 170°C, insbesondere von 140 bis 160°C wird bevorzugt die Herstellung von Alkylchloriden mit einer Anzahl von Kohlenstoffatomen von 11 bis 20 gemäß dem erfindungsgemäßen Verfahren durchgeführt.

Das erfindungsgemäße Verfahren wird vorzugsweise bei Atmosphärendruck durchgeführt.

Der in dem erfindungsgemäßen Verfahren eingesetzte Katalysator kann entweder kommerziell erworben oder in einer vorgeschalteten Reaktion durch Umsetzung von Alkylpyridin oder eines Alkylpyridingemisches mit einem Alkylchlorid bei einer Temperatur von 50 bis 150°C hergestellt werden, wie dies beispielsweise in "Ionic Liquids in Synthesis" (P. Wasserscheid, Wiley-VCH-Verlag, 2003) auf den Seiten 9-12 beschrieben ist. Anschließend wird die Katalysatorphase abgetrennt und durch Wasserdampfdestillation von gelöstem Alkylchlorid befreit. Eine aufwändige Isolierung und Reinigung des Katalysators ist nicht notwendig.

Die Herstellung des Katalysators kann in einem getrennten Verfahren oder in einem vorgeschaltenen Verfahrensschritt des erfindungsgemäßen Verfahrens erfolgen. Bevorzugt wird der in dem erfindungsgemäßen Verfahren eingesetzte Katalysator in einem vorgeschaltenen Verfahrensschritt hergestellt und somit direkt ohne weitere Reinigung eingesetzt. In einer besonderen Ausführungsform des erfindungsgemäßen Verfahrens erfolgt die Herstellung des Katalysators in situ. Es ist jedoch auch möglich in dem erfindungsgemäßen Verfahren kommerziell erhältliche Verbindungen der Strukturen **I** bis **IV** als Katalysatoren einzusetzen.

In einer bevorzugten Ausführungsform des erfindungsgemäßen Verfahrens wird in einem vorgeschalten Verfahrensschritt aus einer Mischung aus Alkyl- und Dialkylpyridinen der Katalysator hergestellt.

Die nachfolgenden Beispiele sollen das erfindungsgemäße Verfahren näher erläutern, ohne dass die Erfindung auf diese Ausführungsform beschränkt sein soll.

### Beispiel 1: Herstellung des Katalysators N-n-Butyl-alkylpyridiniumchlorid

Zu 600 g eines Alkylpyridingemisches werden 675 g n-Butylchlorid zugegeben und für 70 Stunden bei 80°C gerührt. Das Reaktionsgemisch bildet ein Zweiphasensystem, wobei der Katalysator sich in der unteren Phase befmdet. Die obere Phase, die überwiegend das überschüssige n-Butylchlorid aufweist, wird mittels Phasentrennung abgetrennt. In der unteren Phase gelöstes n-Butylchlorid wird durch eine Wasserdampfdestillation entfernt, wenn der Katalysator für die Herstellung von Alkylchloriden verschieden von n-Butylchlorid verwendet wird.

### Beispiel 2: Herstellung des Katalysators N-n-Octyl-alkylpyridiniumchlorid

Zu 1031 g eines Alkylpyridingemisch gemäß Beispiel 1 werden 2180 g n-Octylchlorid zugegeben und für 160 Stunden bei 90°C gerührt. Das Reaktionsgemisch bildet ein Zweiphasensystem, wobei der Katalysator sich in der unteren Phase befindet. Die obere Phase, die überwiegend das überschüssige n-Octylchlorid aufweist, wird mittels Phasentrennung abgetrennt. In der unteren Phase gelöstes n-Octylchlorid wird durch eine Wasserdampfdestillation entfernt, wenn der Katalysator für die Herstellung von Alkylchloriden verschieden von n-Octylchlorid verwendet wird.

### Beispiel 3a: Kontinuierliche Herstellung von n-Butylchlorid

In einem 11 ummantelten Glasreaktor mit einem Glasflügelrührer und Tauchrohr zur Eduktdosierung werden 290,6 g N-n-Butyl-alkylpyridiniumchlorid (hergestellt gemäß Beispiel 1) als Katalysator vorgelegt, auf eine Temperatur von 135°C eingestellt und über das Tauchrohr werden stündlich 50 g n-Butanol und 38 g gasförmiger Chlorwasserstoff zudosiert. Das dabei erzeugte Reaktionsprodukt wird dampfförmig über eine Destillationsbrücke ausgetragen und anschließend kondensiert. Das zweiphasige Reaktionsprodukt wird getrennt und die organische Phase nach bekannten Methoden aufgearbeitet. Nach mehrstufiger Wäsche und Trocknung über Ätznatron wurde Butylchlorid mit einer Reinheit von > 99,5% erhalten.

Analyse:
- Phasenverteilung: obere organische Phase : untere wässrige Phase = 64,7 : 35,3 Gew.-%.
- Umsatz bzgl. n-Butanol: 81 %.

### Vergleichsbeispiel 3b: Kontinuierliche Herstellung von n-Butylchlorid

Die Durchführung ist entsprechend dem Beispiel 3a mit der Ausnahme, dass als Katalysator ein Alkylpyridinhydrochlorid eingesetzt wird.

Analyse:
- Phasenverteilung: obere organische Phase : untere wässrige Phase = 50 : 50 Gew.-%.
- Umsatz bzgl. n-Butanol: 60 %.

### Beispiel 4a: Herstellung von 2-Ethylhexylchlorid im semikontinuierlichen Verfahren

In einem 11 ummantelten Glasreaktor mit einem Glasflügelrührer, einem Tauchrohr zur Eduktdosierung und einem aufgesetzten Wasserauskreiser werden 200 g N-n-Butyl-alkylpyridiniumchlorid (hergestellt gemäß Beispiel 1) als Katalysator vorgelegt und auf eine Temperatur von 135°C eingestellt. Über das Tauchrohr werden 519,8 g 2-Ethylhexanol in 5 h gleichmäßig eingefahren. Während der gesamten Reaktionszeit wird gasförmiger Chlorwasserstoff ebenfalls über das Tauchrohr im leichten stöchiometrischen Überschuss (223 g) zudosiert. Das Reaktionswasser wird azeotrop mit dem Zielprodukt abdestilliert und im Wasserauskreiser als untere Phase abgetrennt, während die obere Phase in den Reaktor zurückgeführt wird. Nach einer Gesamtreaktionszeit von 22 h beträgt der Umsatz bezogen auf das 2-Ethylhexanol 99,3%.

### Vergleichsbeispiel 4b: Herstellung von 2-Ethylhexylchlorid im semikontinuierlichen Verfahren

Die Durchführung ist entsprechend dem Beispiel 4a mit der Ausnahme, dass als Katalysator ein Alkylpyridinhydrochlorid eingesetzt wird. Nach einer Gesamtreaktionszeit von 22 h beträgt der Umsatz bezogen auf das 2-Ethylhexanol 81,9% und nach einer Gesamtreaktionszeit von 34 h beträgt er 93 %.

### Beispiel 5a: Kontinuierliche Herstellung von n-Octylchlorid in einer Kaskadenfahrweise

Zwei in Reihe geschaltete 1 1 Glasreaktoren werden mit einem seitlich angebrachtem Überlauf verbunden. Der Überlauf ist so konstruiert und angebracht, dass nur die obere Produktphase in den Folgereaktor überführt wird und die untere Katalysatorphase im Reaktor verbleibt. In beiden Reaktoren wird 460 g N-n-Octyl-alkylpyridiniumchlorid (hergestellt gemäß Beispiel 2) als Katalysator bis zum Überlauf vorgelegt und auf eine Temperatur von 135 - 147°C eingestellt. In den ersten Reaktor wird n-Octanol und gasförmiger Chlorwasserstoff (10-50 g/h je nach n-Octanol-Zulauf) kontinuierlich über ein Tauchrohr zudosiert. In den zweiten Reaktor wird die obere organische Phase des ersten Reaktors kontinuierlich überführt und ebenfalls gasförmigen Chlorwasserstoff (5-15 g/h) über ein Tauchrohr kontinuierlich eingeleitet. Das bei der Reaktion gebildete Reaktionswasser wird in beiden Reaktoren azeotrop über einen Wasserauskreiser ausgetragen, wobei die obere n-Octylchlorid-Phase in den jeweiligen Reaktor zurückgeführt wird. Chlorwasserstoff wird in beiden Reaktoren in einem leichten stöchiometrischen Überschuss zugegeben. Abhängig von den Zulaufmengen an n-Octanol beim ersten Reaktor werden folgende Umsätze bezogen auf das n-Octanol erzielt:

| Zulauf an n-Octanol beim 1. Reaktor *(in g*/*h)* | *Umsatz an n-Octanol im 1. Reaktor (in %)* | *Umsatz an n-Octanol im 2. Reaktor (in %)* |
|---|---|---|
| 26,5 | 99,3 | >99,9 |
| 43 | 98,7 | >99,9 |
| 66,4 | 97,7 | >99,9 |
| 93,7 | 95,8 | >99,8 |
| 108 | 94,6 | 99,8 |

### Vergleichsbeispiel 5b: Kontinuierliche Herstellung von n-Octylchlorid in einer Kaskadenfahrweise

Die Durchführung ist entsprechend dem Beispiel 5a mit der Ausnahme, dass als Katalysator ein Alkylpyridinhydrochlorid eingesetzt wird. Abhängig von den Zulaufmengen an n-Octanol beim ersten Reaktor werden folgende Umsätze bezogen auf das n-Octanol erzielt:

| Zulauf an n-Octanol beim 1. Reaktor *(in g*/*h)* | Umsatz an n-Octanol im 1. Reaktor *(in %)* | Umsatz an n-Octanol im 2. Reaktor *(in %)* |
|---|---|---|
| 34 | 94,8 | 99,8 |

### Beispiel 6a: Herstellung von n-Tetradecylchlorid im semikontinuierlichen Verfahren

In einem 1 1 ummantelten Glasreaktor mit einem Glasflügelrührer, einem Tauchrohr zur Eduktdosierung und einem aufgesetzten Wasserauskreiser werden 200 g N-n-Octyl-alkylpyridiniumchlorid (hergestellt gemäß Beispiel 2) als Katalysator vorgelegt und auf eine Temperatur von 150°C eingestellt. Über das Tauchrohr werden 243 g n-Tetradecanol gleichmäßig in 4,5 h eingefahren. Während der gesamten Reaktionszeit wird gasförmiger Chlorwasserstoff im leichten Überschuss über ein Tauchrohr zudosiert, während das gebildete Reaktionswasser destillativ aus der Reaktionsmischung entfernt wird. Die Durchführung der Reaktion und Aufarbeitung erfolgt analog zu Beispiel 4a. Nach einer Gesamtreaktionszeit von 7,5 h beträgt der Umsatz bzgl. n-Tetradecanol 99,5 %.

### Vergleichsbeispiel 6b: Herstellung von n-Tetradecylchlorid im semikontinuierlichen Verfahren

Die Durchführung ist entsprechend dem Beispiel 6a mit der Ausnahme, dass als Katalysator ein Alkylpyridinhydrochlorid eingesetzt wird. Nach einer Gesamtreaktionszeit von 7,5 h beträgt der Umsatz bzgl. n-Tetradecanol 87,2 % und nach einer Gesamtreaktionszeit von 15,5 h beträgt er 99,2 %.

### Beispiel 7a: Herstellung von n-Octadecylchlorid im semikontinuierlichen Verfahren

In einem 1 1 ummantelten Glasreaktor mit einem Glasflügelrührer und Tauchrohr zur Eduktdosierung werden 220 g N-n-Octyl-alkylpyridiniumchlorid (hergestellt gemäß Beispiel 2) vorgelegt und auf eine Temperatur von 150°C eingestellt. Über ein Tauchrohr werden 447 g n-Octadecanol gleichmäßig über 5 Stunden eingefahren. Gasförmiger Chlorwasserstoff wird während der gesamten Reaktionszeit im leichten stöchiometrischen Überschuss über ein Tauchrohr zudosiert und gleichzeitig das gebildete Reaktionswasser destillativ entfernt. Die Durchführung der Reaktion und die Aufarbeitung der Reaktionsmischung erfolgt analog zu Beispiel 4a. Nach einer Gesamtreaktionszeit von 8 h (inklusive von 3 Stunden Nachreaktion) beträgt der Umsatz bezogen auf n-Octadecanol 99,8 %.

### Beispiel 7b: Herstellung von n-Octadecylchlorid im semikontinuierlichen Verfahren

Die Durchführung ist entsprechend dem Beispiel 7a mit der Ausnahme, dass als Katalysator ein Alkylpyridinhydrochlorid eingesetzt wird. Nach einer Gesamtreaktionszeit von 8 h beträgt der Umsatz bzgl. dem n-Octadecanol 62,7 % und nach einer Gesamtreaktionszeit von 39 h beträgt er 99,4 %.

## Patentansprüche

1. Verfahren zur Herstellung von Alkylchloriden durch Umsetzung der entsprechenden Alkohole mit gasförmigem Chlorwasserstoff in Gegenwart eines Katalysators,
**dadurch gekennzeichnet,**
**dass** ein Katalysator, der zumindest eine Verbindung gemäß der Struktur: 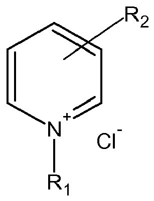 mit
R₁: lineare Alkylgruppe mit einer Anzahl an Kohlenstoffatomen von 1 bis 20,
R₂: Alkyl-Gruppe mit einer Anzahl an Kohlenstoffatomen von 1 bis 20,
aufweist,
in Form einer Mischung eingesetzt wird, die verschiedene Isomere an mono-alkylierten und/oder mehrfach alkylierten N-Alkylpyridiniumchloriden aufweist, wobei sich die mehrfach alkylierte N-Alkylpyridiniumchloride durch mehrere Substituenten vom Typ R₂ am Stickstoffheterocyclus auszeichnen.

2. Verfahren gemäß Anspruch 1,
**dadurch gekennzeichnet,**
**dass** das Verfahren kontinuierlich durchgeführt wird.

3. Verfahren gemäß Anspruch 2,
**dadurch gekennzeichnet,**
**dass** das Verfahren in mehreren Reaktoren, die in Form einer Kaskade mit einander verbunden sind, durchgeführt wird.

4. Verfahren gemäß zumindest einem der Ansprüche 1 bis 3,
**dadurch gekennzeichnet,**
**dass** Alkohole, die von 3 bis 20 Kohlenstoffatome aufweisen, eingesetzt werden.

5. Verfahren gemäß Anspruch 4,
**dadurch gekennzeichnet,**
**dass** ein linearer Alkohol mit einer Hydroxy-Gruppe, ausgewählt aus n-Propanol, n-Butanol, n-Hexanol, n-Octanol, n-Tetradecanol, n-Octadecanol, eingesetzt wird.

6. Verfahren gemäß Anspruch 4,
**dadurch gekennzeichnet,**
**dass** ein verzweigter Alkohol mit einer Hydroxy-Gruppe, ausgewählt aus iso-Propanol, iso-Butanol, sec-Butanol, 2-Ethylhexanol, eingesetzt wird.

7. Verfahren gemäß Anspruch 4,
**dadurch gekennzeichnet,**
**dass** ein Alkohol mit zwei Hydroxy-Gruppen, ausgewählt aus 1,6-Hexandiol, 1,8-Octandiol und 1,10-Decandiol, eingesetzt wird.

8. Verfahren gemäß zumindest einem der Ansprüche 1 bis 7,
**dadurch gekennzeichnet,**
**dass** das Reaktionsprodukt, dampfförmig aus dem Reaktor ausgetragen wird.

9. Verfahren gemäß zumindest einem der Ansprüche 1 bis 8,
**dadurch gekennzeichnet,**
**dass** das Reaktionsprodukt über einen seitlich angebrachten Überlauf abgetrennt wird.

10. Verfahren gemäß zumindest einem der Ansprüche 1 bis 9,
**dadurch gekennzeichnet,**
**dass** das Verfahren bei einer Temperatur von 60 bis 160°C durchgeführt wird.

11. Verfahren gemäß zumindest einem der Ansprüche 1 bis 10,
**dadurch gekennzeichnet**,
der eingesetzte Katalysator in einem vorgeschaltenen Verfahrensschritt hergestellt wird.

## Claims

1. Process for preparing alkyl chlorides by reacting the corresponding alcohols with gaseous hydrogen chloride in the presence of a catalyst,
**characterized in that**
a catalyst which comprises at least one compound of the structure: 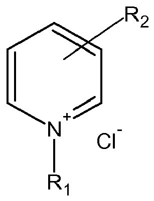 where
R₁: linear alkyl group having a number of carbon atoms of from 1 to 20,
R₂: alkyl group having a number of carbon atoms of from 1 to 20,
is used in the form of a mixture which comprises different isomers of monoalkylated and/or polyalkylated N-alkylpyridinium chlorides, the polyalkylated N-alkylpyridinium chlorides featuring a plurality of substituents of the R₂ type on the nitrogen heterocycle.

2. Process according to Claim 1,
**characterized in that**
the process is carried out continuously.

3. Process according to Claim 2,
**characterized in that**
the process is carried out in a plurality of reactors which are connected to one another in the form of a battery.

4. Process according to at least one of Claims 1 to 3,
**characterized in that**
alcohols which have from 3 to 20 carbon atoms are used.

5. Process according to Claim 4,
**characterized in that**
a linear alcohol having one hydroxyl group, selected from n-propanol, n-butanol, n-hexanol, n-octanol, n-tetradecanol, n-octadecanol, is used.

6. Process according to Claim 4,
**characterized in that**
a branched alcohol having one hydroxyl group, selected from isopropanol, isobutanol, sec-butanol, 2-ethylhexanol, is used.

7. Process according to Claim 4,
**characterized in that**
an alcohol having two hydroxyl groups, selected from 1,6-hexanediol, 1,8-octanediol and 1,10-decanediol, is used.

8. Process according to at least one of Claims 1 to 7,
**characterized in that**
the reaction product is discharged from the reactor in vapor form.

9. Process according to at least one of Claims 1 to 8,
**characterized in that**
the reaction product is removed via a laterally attached overflow.

10. Process according to at least one of Claims 1 to 9,
**characterized in that**
the process is carried out at a temperature of from 60 to 160°C.

11. Process according to at least one of Claims 1 to 10,
**characterized in that**
the catalyst used is prepared in a preceding process step.

## Revendications

1. Procédé de fabrication de chlorures d'alkyle par mise en réaction des alcools correspondants avec du chlorure d'hydrogène gazeux en présence d'un catalyseur, **caractérisé en ce qu'**un catalyseur comprenant au moins un composé selon la structure : 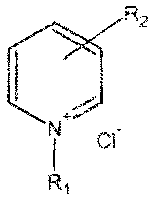 avec
R₁ : groupe alkyle linéaire ayant un nombre d'atomes de carbone de 1 à 20,
R₂ : groupe alkyle ayant un nombre d'atomes de carbone de 1 à 20,
est utilisé sous la forme d'un mélange, qui comprend différents isomères de chlorures de N-alkylpyridinium monoalkylés et/ou polyalkylés, les chlorures de N-alkylpyridinium polyalkylés étant **caractérisés par** plusieurs substituants de type R₂ sur l'hétérocycle azoté.

2. Procédé selon la revendication 1, **caractérisé en ce que** le procédé est réalisé en continu.

3. Procédé selon la revendication 2, **caractérisé en ce que** le procédé est réalisé dans plusieurs réacteurs, qui sont reliés les uns avec les autres sous la forme d'une cascade.

4. Procédé selon au moins l'une quelconque des revendications 1 à 3, **caractérisé en ce que** des alcools comprenant 3 à 20 atomes de carbone sont utilisés.

5. Procédé selon la revendication 4, **caractérisé en ce qu'**un alcool linéaire contenant un groupe hydroxy, choisi parmi le n-propanol, le n-butanol, le n-hexanol, le n-octanol, le n-tétradécanol, le n-octadécanol, est utilisé.

6. Procédé selon la revendication 4, **caractérisé en ce qu'**un alcool ramifié contenant un groupe hydroxy, choisi parmi l'isopropanol, l'isobutanol, le sec-butanol, le 2-éthylhexanol, est utilisé.

7. Procédé selon la revendication 4, **caractérisé en ce qu'**un alcool contenant deux groupes hydroxy, choisi parmi le 1,6-hexanediol, le 1,8-octanediol et le 1,10-décanediol, est utilisé.

8. Procédé selon au moins l'une quelconque des revendications 1 à 7, **caractérisé en ce que** le produit de réaction est déchargé sous forme gazeuse du réacteur.

9. Procédé selon au moins l'une quelconque des revendications 1 à 8, **caractérisé en ce que** le produit de réaction est séparé par un trop-plein disposé latéralement.

10. Procédé selon au moins l'une quelconque des revendications 1 à 9, **caractérisé en ce que** le procédé est réalisé à une température de 60 à 160 °C.

11. Procédé selon au moins l'une quelconque des revendications 1 à 10, **caractérisé en ce que** le catalyseur utilisé est fabriqué lors d'une étape de procédé en amont.
